# EUROPÄISCHE PATENTSCHRIFT

(11) **EP 2 822 514 B1**
(45) Veröffentlichungstag und Bekanntmachung des Hinweises auf die Patenterteilung: **02.05.2018**
(21) Anmeldenummer: 13710779.3
(22) Anmeldetag: 07.02.2013
(51) Int. Cl.: A61F 2/89, A61F 2/915

(54) **GEFÄSSSTÜTZE HOHER FLEXIBILITÄT MIT SOLLBRUCHSTELLE**
HIGHLY FLEXIBLE STENT HAVING A PREDETERMINED BREAKING POINT
TUTEUR VASCULAIRE À HAUTE FLEXIBILITÉ PRÉSENTANT UN POINT DE RUPTURE

(30) Priorität: 03.03.2012 DE 202012002340 U
(43) Veröffentlichungstag der Anmeldung: 14.01.2015
(73) Patentinhaber: Osypka, Peter, 79639 Grenzach-Wyhlen (DE)
(72) Erfinder: Osypka, Peter, 79639 Grenzach-Wyhlen (DE)
(86) Internationale Anmeldenummer: PCT/DE2013/000067
(87) Internationale Veröffentlichungsnummer: WO 2013/131501

(56) Entgegenhaltungen:
- EP-A1- 1 563 806
- EP-A1- 1 958 598
- WO-A1-2007/013102
- DE-U1-202011 003 403
- US-A1- 2006 069 424

## Beschreibung

Die Erfindung betrifft eine implantierbare Gefäßstütze, die als Stützhülse im Bereich einer insbesondere mittels Ballonkatheter ausweitbaren Arterienverengung einsetzbar ist, wobei die Wandung der Gefäßstütze dehnbar ist und in radialer Richtung dadurch ausweitbar ist, dass sie zumindest bereichsweise aus bleibend verformbaren Metallstegen gebildet ist und bei Überschreiten einer gewissen radialen Ausdehnung die Metallstege infolge einer eingebauten Sollbruchstelle sich öffnen.
Gefäßstützen dieser Art werden besonders bei der Behandlung der Pulmonalstenose bei Kindern benötigt.

Die Möglichkeiten des Ausweitens in radialer Richtung sind je nach Modell der Gefäßstütze beschränkt. Vor altem in solchen Fällen, in denen größere Ausweitungsmöglichkeiten in mehreren Schritten erwünscht sind, wie dies bei Kindern mit einer Pulmonalstenose der Fall ist, können Schwierigkeiten auftreten. Vermeidet man eine sofortige Operation und implantiert diesen Säuglingen einen normalen Stent, so bildet dieser nach ein paar Jahren wachsender Gefäße selbst eine Stenose.

Gefäßstützen in dieser Art sind in vielfältiger Form bereits bekannt.
DE 101 05 160 B4 beschreibt eine ausweitbare Gefäßstütze. Die Gefäßstütze hat in Längsrichtung eine Unterbrechung. Beidseits der Unterbrechung sind Ösen vorgesehen, die durch einen absorbierbaren Faden zusammengehalten werden, sodass die Gefäßstütze zunächst geschlossen ist. Nach Entfernen oder Auflösen des Fadens kann sich die Gefäßstütze öffnen, sodass eine weitere Ausweitung der Gefäßstütze möglich wird.

DE 10 2004 027 108 B4 beschreibt eine Gefäßstütze, die in zwei Schritten ausweitbar ist Während einer ersten Dehnung ist ein Wandbereich durch eine Halterung aus biologisch abbaubarem Material gegen Dehnung blockiert. Nachdem sich die Halterung aufgelöst hat ist eine weitere Dehnung möglich.

DE 101 03 000 A1 beschreibt eine radial ausweitbare Gefäßstütze. Die gitterförmige Gefäßstütze besteht aus mindestens zwei rohrförmigen Teilstücken mit einer zickzackförmigen Ringstruktur mit einer oder mehreren Wachstumsfugen, die mithilfe von resorbierbaren chirurgischem Nahtmaterial zeitlich begrenzt fest miteinander verbunden sind und ein weiteres Wachstum des Gefäßes erlauben, was auch durch eine erneute Ballondilatation unterstützt werden kann.

Die europäische Patentanmeldung EP 1958598 offenbart eine implantierbare Gefäßstütze, die in radialer Richtung dehnbar ist. Die Gefäßstütze besteht aus mehreren Segmenten, die durch Stege miteinander verbunden sind. Die Stege können auch eine Sollbruchstelle aufweisen. Die Segmente bestehen aus Metallstegen, die mindestens eine Sollbruchstelle aufweisen. Die Gefäßstütze weist nur eine geringe Flexibilität in Längsrichtung auf.

Aus DE 10 2008 045 039 A1 ist eine Gefäßstütze mit einer Sollbruchstelle bekannt, die aber durch ihre spiralförmige Konstruktion bei gleichzeitiger radialer Pulsation der Gefäße, ausgelöst durch den Herzschlag, in ihrer radialen Ausweitung inkonstante Sollbrucheigenschaften aufweist.

Eine weitere Gefäßstütze ist aus US 5 591 223 bekannt. Hier erfolgt eine erweiterte radiale Öffnung durch ein unkontrolliertes Reißen und nicht durch eine definierte Sollbruchstelle.

Das deutsche Gebrauchsmuster 202011003403 beschreibt eine Gefäßstütze die aus röhrenförmigen Segmenten besteht, wobei die Segmente aus Metallstegen bestehen, und wobei mindestens einer der Metallstege innerhalb des Segments eine Sollbruchstelle aufweist die durch eine geschlitzte Öse gebildet wird.

Was den bisherigen Gefäßstützen fehlt, ist eine hohe Flexibilität zwischen den einzelnen Segmenten kombiniert mit der Möglichkeit, dass die Wandung der Gefäßstütze dehnbar (ausweitbar) ist. Gerade bei Kindern im Säuglingsalter kann eine zu große Steifigkeit der Gefäßstütze zu einer Perforation des Gefäßes führen. Durch das Wachstum der Kinder vergrößern sich auch die Gefäße gegenüber dem Zeitpunkt der Implantation, sodass der dem Kleinkind implantierte Stent gegen einen größeren Stent ausgetauscht werden muss.

Ferner bereiten die oben beschriebenen ausweitbaren Gefäßstützen aufgrund ihrer metallischen Struktur im geschlossenen Zustand Probleme, wenn sich der Patient einer Magnetresonanztomographie, abgekürzt MRT unterziehen muss.

Es besteht daher die Aufgabe eine Gefäßstütze der eingangs definierter Art zu schaffen, die eine große radiale Dehnbarkeit erlaubt und gleichzeitig eine hohe Flexibilität besitzt. Ferner darf die Gefäßstütze die Magnetfelder und damit die Bildgebung einer MRT-Aufnahme nicht stören.

Zur Lösung dieser Aufgabe wird eine implantierbare Gefäßstütze vorgeschlagen, die als Stützhülse im Bereich einer insbesondere mittels Ballonkatheter ausweitbaren Arterienverengung einsetzbar ist, wobei die Wandung der Gefäßstütze dehnbar ist und in radialer Richtung dadurch ausweitbar ist, dass die Wandung zumindest bereichsweise aus bleibend verformbaren Metallstegen gebildet ist, dadurch gekennzeichnet, dass die Gefäßstütze aus mehr als einem röhrenförmigen Segment (10) besteht, wobei die Segmente (10) aus Metallstegen (2) bestehen und wobei je zwei benachbarte Segmente (10) durch mindestens ein Ösenpaar (6) und (7) miteinander verbunden sind, wobei die Ösen (6) und (7) durch Einbettung in einen Kunststoff miteinander verbunden sind und so die Segmente (10) zusammengehalten werden und wobei mindestens einer der Metallstege (2) im mittleren Segmentabschnitt (40) eine Sollbruchstelle aufweist, die dadurch entsteht dass jeweils zwei Stegabschnitte (3),(4) Ösen (11), (12) aufweisen, die ebenfalls durch Einbettung in einen Kunststoff miteinander derart verbunden sind, dass bei Überschreiten einer definierten radialen Ausdehnung, die Metallstege (2) infolge der Sollbruchstelle sich öffnen.

Welche Vorteile haben die technischen Merkmale?
Der wichtigste Vorteil dieser Erfindung liegt darin, dass mithilfe einer hochflexiblen Gefäßstütze und mit einer durch einen weichen Kunststoff gebildeten Sollbruchstelle eine spätere Operation bei Kindern mit einer angeborenen Pulmonalstenose, Aortenisthmusstenose oder anderen Indikationen des Gefäßsystems vermieden werden kann.

Die hohe Flexibilität ergibt sich durch die Verbindung der Segmente (10) über die in Kunststoff eingebetteten Ösen (6) und (7),

Wachsen oder verändern sich die Gefäße nach erfolgter Implantation besteht die Möglichkeit die Gefäßstütze mithilfe eines Ballons erneut zu dehnen. Diese Möglichkeit ergibt sich durch die Verbindung der Metallstege über die in Kunststoff eingebetteten Ösen (11) und (12), welche eine Sollbruchstelle bilden.

Die Ösen (11) und (12) aus nichtleitendem Kunststoff sorgen dafür, dass die metallische Wandung der Gefäßstütze unterbrochen ist, sodass sich während einer MRT-Aufnahme keine die Bildgebung störenden Induktionsströme bilden können.

Die in Kunststoff eingebetteten Ösen haben weiterhin den Vorteil, dass im Kunststoff ein Reservoir geschaffen werden kann um Medikamente freizusetzen und damit eine Art von Drug Eluting Stent entsteht.

Der Begriff "bleibend verformbare Metallstege" bedeutet dass die Gefäßstütze im ausgeweiteten Zustand in radialer Richtung quer zur Längsachse der Metallstege dauerhaft ausgeweitet bleibt.
Die Erfindung betrifft auch eine Gefäßstütze wie in Anspruch 1 definiert zur Anwendung in einem Verfahren zur Behandlung der Pulmonalstenose oder Aortenistmusstenose bei Kindern.

### Darstellung der Abbildungen

Weitere Einzelheiten, Merkmale und Vorteile der Erfindung lassen sich im folgenden Beschreibungsteil entnehmen, in dem anhand der Zeichnungen die Erfindung näher erläutert wird. Die Abbildungen zeigen in schematischer Darstellung in
**Fig. 1** eine beispielhafte Darstellung der implantierbaren Gefäßstütze.
**Fig. 2** eine beispielhafte Darstellung von einem Teil der implantierbaren Gefäßstütze in zwei-dimensionaler Darstellung.
**Fig. 3** eine detaillierte Darstellung der in Kunststoff eingebetteten Ösen.

### Beschreibung der Abbildungen:

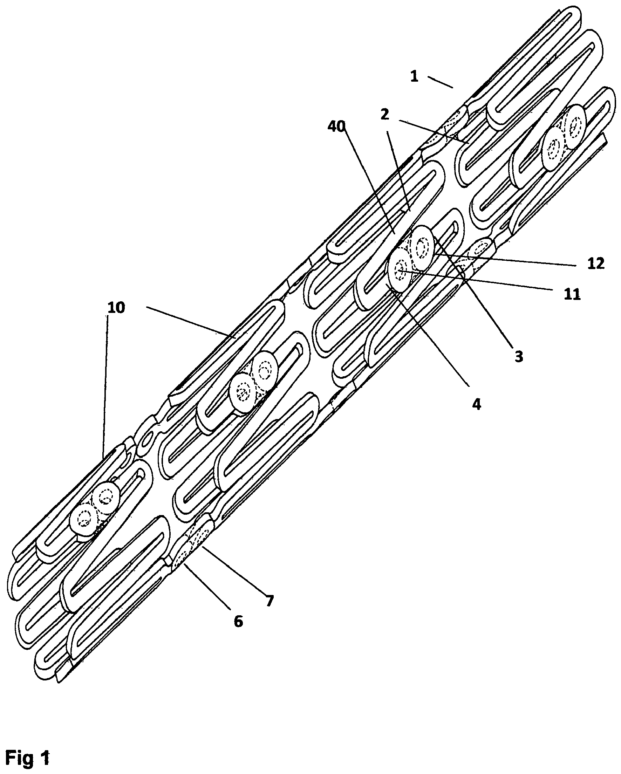

**Fig. 1** zeigt eine beispielhafte Darstellung der implantierbaren Gefäßstütze (1).
Die Gefäßstütze besteht aus röhrenförmigen Segmenten (10), Mindestens zwei Segmente müssen vorhanden sein. Die Abbildung zeigt 4 Segmente (10). Üblicherweise besteht die Gefäßstütze aus 3-5 Segmenten.
Die Segmente bestehen aus Metallstegen (2). Die Stege (2) der Gefäßstütze sind so geformt, dass sie eine möglichst große radiale Expansion ermöglichen. Die Stege (2) zeigen vorzugsweise einen sinusförmigen Verlauf und bestehen beispielsweise aus Edelstahl oder aus Nitinol.
In den Abschnitten oder Segmenten (10), die die radiale Expansion bestimmen, weisen jeweils zwei Stegabschnitte (3) und (4) im mittleren Segmentabschnitt (40) Ösen (11, 12) auf, die durch Einbettung in einen nicht leitenden Kunststoff miteinander verbunden sind. Die Segmente sind durch mindestens ein Ösenpaar (6) und (7) miteinander verbunden. Die Ösen (6) und (7) durch Kunststoffeinbettung miteinander verbunden sind.
Fig: 1 zeigt eine Ausführungsform wobei die Segmente (10) durch zwei Verbindungsstellen, gebildet durch die Ösen (6) und (7), miteinander verbunden sind.

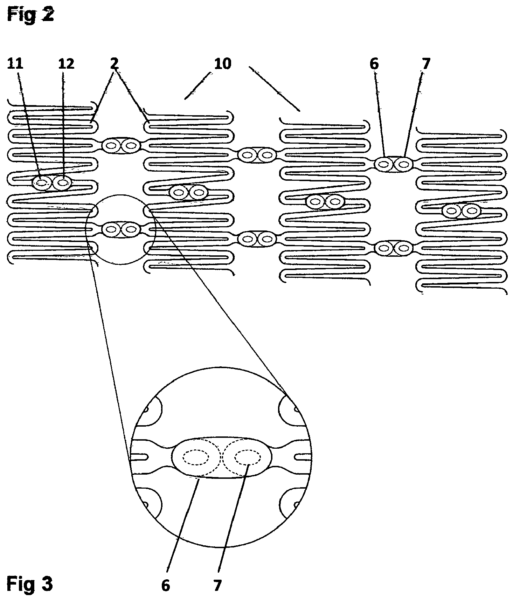

**Fig. 2** zeigt in einer beispielhaften Darstellung einen Teil der implantierbaren Gefäßstütze in zwei-dimensionaler Ausführung. Man erkennt, dass die Ösen (6) und (7) und die Ösen (11) und (12) eng beieinander liegen und in Kunststoff eingebettet sind. Die Abmessungen und die Materialeigenschaften der Ösen (11) und (12) und des sie umgebenden Kunststoffes bestimmen im Wesentlichen die gewünschte Sollbruchstelle. Die Abmessungen und die Materialeigenschaften der Ösen (6) und (7) und des sie umgebenden Kunststoffs bestimmen im Wesentlichen die gewünschte Flexibilität der einzelnen Segmente (10).
Die Ösen (6), (7), (11) und (12) bestehen vorzugsweise aus demselben Material wie die Stege (2). Der Kunststoff besteht vorzugsweise aus Silikon oder aus Polyurethan und löst sich im Körper nicht auf. Besonders bevorzugt ist Silikon.

Die Kunststoffeinbettung kann für die Ösen (6) und (7) und für die Ösen (11) und (12) dieselbe sein oder auch verschieden sein. Beispielsweise können die Ösen (6) und (7) in Silikon eingebettet sein und die Ösen (11) und (12) in Polyurethan. Vorzugsweise ist der Kunststoff identisch.

Die Ösen (6), (7) und (11) und (12) werden jeweils nebeneinanderliegend in Kunststoff eingebettet. Es ist aber auch möglich, dass zwischen den Ösen (6), (7) und zwischen den Ösen (11) und (12) bei der Einbettung ein Abstand vorhanden ist.

**Fig. 3** eine detaillierte Darstellung der implantierbaren Gefäßstütze.

## Patentansprüche

1. Implantierbare Gefäßstütze die als Stützhülse im Bereich einer insbesondere mittels Ballonkatheter ausweitbaren Arterienverengung einsetzbar ist, wobei die Wandung der Gefäßstütze dehnbar ist und in radialer Richtung dadurch ausweitbar ist, dass die Wandung zumindest bereichsweise aus bleibend verformbaren Metallstegen gebildet ist, wobei die Gefäßstütze aus mehr als einem röhrenförmigen Segment (10) besteht, wobei die Segmente (10) aus Metallstegen (2) bestehen und wobei mindestens einer der Metallstege (2) im mittleren Segmentabschnitt (40) eine Sollbruchstelle aufweist, **dadurch gekennzeichnet, dass** die Sollbruchstelle dadurch entsteht dass jeweils zwei Stegabschnitte (3),(4) Ösen (11), (12) aufweisen, die durch Einbettung in einen Kunststoff miteinander derart verbunden sind, dass bei Überschreiten einer definierten radialen Ausdehnung, die Metallstege infolge der Sollbruchstelle sich öffnen und wobei je zwei benachbarte Segmente (10) durch mindestens ein Ösenpaar (6) und (7) miteinander verbunden sind, wobei die Ösen (6) und (7) durch Einbettung in einen Kunststoff miteinander verbunden sind und so die Segmente (10) zusammengehalten werden.

2. Gefäßstütze nach Anspruch 1 **dadurch gekennzeichnet, dass** der Kunststoff Silikon oder Polyurethan, vorzugsweise Silikon ist.

3. Gefäßstütze nach Anspruch 1 oder 2, **dadurch gekennzeichnet, dass** die Verbindung der Ösen (6) und (7) und der Ösen (11) und (12) aus unterschiedlichen Kunststoffen besteht.

4. Gefäßstütze nach Anspruch 1 **dadurch gekennzeichnet, dass** zwischen den Ösen (6), (7) und zwischen den Ösen (11) und (12) bei der Einbettung ein Abstand vorhanden ist.

5. Gefäßstütze nach Anspruch 1, **dadurch gekennzeichnet, dass** die Kunststoff Verbindung zwischen den Ösen Medikamente enthält.

## Claims

1. Implantable stent which can be used to stabilize a narrowed artery which is dilatable by means of a balloon catheter; the stent comprises a stent body which is deformable and radially expandable because the stent body is at least partially formed of deformable metallic strut members, wherein the stent body has more than one tubular segment (10), said segments (10) consist of metallic strut members (2) and wherein at least one of the metallic strut members (2) has a predetermined breaking point in its middle segment portion (40), **characterized in that** the predetermined breaking point is formed by two interconnecting strut sections (3) and (4) having eyelets (11) and (12) that are embedded in plastic such that the strut sections open when they exceed a defined radial extension due to the predetermined breaking point and wherein at least two adjacent segments (10) are interconnected by eyelets (6) and (7) that are embedded in plastic so that the segments (10) are held together.

2. Stent according to claim 1 **characterized in that** plastic is silicone or polyurethane, preferably silicone.

3. Stent according to claim 1 or 2 **characterized in that** the plastic used between eyelets (6) and (7) is different to the plastic used between eyelets (11) and (12).

4. Implantable stent according to claim 1 **characterized in that** there is a gap between eyelets (6) and (7) and between eyelets (11) and (12) when being embedded.

5. Implantable stent according to claim 1 **characterized in that** the plastic compound between the eyelets contains a drug.

## Revendications

1. Endoprothèse vasculaire implantable destinée à stabiliser une artère rétrécie qui est dilatable par un cathéter à ballonnet; l'endoprothèse a un corps étant apte à s'étendre radialement parce que le corps d'endoprothèse est formé au moins partiellement d'éléments entretoises en métal déformable, le corps d'endoprothèse est formé de plusieurs segments tubulaires (10), les segments (10) sont constitués en métal déformable (2); au moins l'une des entretoises en métal (2) a un point de rupture prédéterminé dans la partie de segment intermédiaire (40), **caractérisé en ce que** le rupture prédéterminé est formé par deux sections d'entretoises (3), (4) ayant une paire d'oeillets (11) et (12) reliés l'un à l'autre en utilisant du plastique de sorte que lorsqu'une dilatation radiale définie est dépassée les entretoises en métal (2) s'ouvrent à cause du point de rupture prédéterminé; et au moins deux segments adjacents (10) sont reliés par une paire d'oeillets (6) et (7), les oeillets (6) et (7) étant reliés l'un à l'autre en utilisant du plastique et ainsi les segments (10) sont maintenus ensemble.

2. Endoprothèse selon la revendication 1, **caractérisé en ce que** la matière plastique est du silicone ou du polyuréthane, de préférence de silicone.

3. Endoprothèse selon la revendication 1 ou 2, **caractérisé en ce que** la matière plastique utilisée pour relier les oeillets (6) et (7) est différent de la matière plastique utilisée pour relier les oeillets (11) et (12).

4. Endoprothèse selon la revendication 1, **caractérisé en ce que** il y a un écart entre les oeillets (6) et (7) et les oeillets (11) et (12) lorsque les oeillets sont reliés.

5. Endoprothèse selon la revendication 1, **caractérisé en ce que** la matière plastique utilisée pour relier les oeillets contient un médicament.
